# EUROPEAN PATENT APPLICATION

(11) **EP 3 202 424 A1**
(43) Date of publication of application: **09.08.2017**
(21) Application number: 17151823.6
(22) Date of filing: 17.01.2017
(51) Int. Cl.: A61K 47/62, A61K 47/69, A61K 31/704, A61P 35/00, A61K 49/18, A61K 51/12

(54) **KIT FOR FORMULATION OF LIPOSOMAL DOXORUBICIN MODIFIED WITH BIOACTIVE PEPTIDES FOR SELECTIVE TARGETING OF RECEPTORS OVEREXPRESSED BY CANCER CELLS**

(30) Priority: 21.01.2016 IT UB20160191
(71) Applicant: Invectors S.r.l., 80122 Napoli (IT)
(72) Inventor: ACCARDO, Antonella, 80059 Torre del Greco (NA) (IT); RINGHIERI, Paola, 84018 Scafati (SA) (IT); MORELLI, Giancarlo, 80135 Napoli (NA) (IT)
(74) Representative: Bianchetti Bracco Minoja S.r.l.

(57) **Abstract**

The invention concerns the obtainment of liposomal doxorubicin in which liposomes are externally modified with a targeting peptide able to drive liposomal doxorubicin in a selective way on membrane receptors over expressed in tumours.

The invention is based on a kit containing a first vial filled with a sterile, translucent, red dispersion of the liposomal doxorubicin drug; a second vial filled with a modified phospholipid with a reactive function (compound 1) such as DSPE-Peg-maleimide in lyophilized form; and a third vial filled with a peptide modified with an appropriate reactive function (compound 2), in a 1:1 stoichiometric amount respect to compound 1 and in lyophilized form.

The kit object of the present invention could also contain an additional component, in a fourth vial, based on the targeting peptide modified with a chelating agent able to complex radioactive metal ions for diagnostic and imaging purposes. In this case patients to be treated with the peptide modified liposomal doxorubicin could be selected on the basis of the over expression of the targeting receptors.

SEQUENCE LISTING

<110>  INVECTORS srl

<120>  Kit per la preparazione di doxorubicina liposomiale
       funzionalizzata con peptidi per il target selettivo di recettori
       sovra espressi da cellule tumorali

<130>  10855M

<160>  17

<170>  PatentIn version 3.5

<210>  1
<211>  8
<212>  PRT
<213>  Artificial Sequence

<220>
<223>  Analogs of C-terminal bombesin fragments


<220>
<221>  MISC_FEATURE
<222>  (8)..(8)
<223>  Xaa is Nle

<400>  1

Gln Trp Ala Val Gly His Leu Xaa
1               5


<210>  2
<211>  8
<212>  PRT
<213>  Artificial Sequence

<220>
<223>  Analogs of C-terminal bombesin fragments


<220>
<221>  MISC_FEATURE
<222>  (7)..(7)
<223>  Xaa is Cha

<220>
<221>  MISC_FEATURE
<222>  (8)..(8)
<223>  Xaa is Nle

<400>  2

Gln Trp Ala Val Gly His Xaa Xaa
1               5


<210>  3
<211>  8
<212>  PRT
<213>  Artificial Sequence

<220>
<223>  Analogs of C-terminal bombesin fragments


<220>
<221>  MISC_FEATURE
<222>  (7)..(7)
<223>  Xaa is Sta

<400>  3

Gln Trp Ala Val Gly His Xaa Leu
1               5


<210>  4
<211>  8
<212>  PRT
<213>  Artificial Sequence

<220>
<223>  Analogs of C-terminal bombesin analogs


<220>
<221>  MISC_FEATURE
<222>  (5)..(5)
<223>  Xaa is NMe Gly

<220>
<221>  MISC_FEATURE
<222>  (7)..(7)
<223>  Xaa is Sta

<400>  4

Gln Trp Ala Val Xaa His Xaa Leu
1               5


<210>  5
<211>  9
<212>  PRT
<213>  Artificial Sequence

<220>
<223>  Analogs of C-terminal bombesin fragments


<220>
<221>  MISC_FEATURE
<222>  (1)..(1)
<223>  Xaa is D-Phe

<220>
<221>  MISC_FEATURE
<222>  (8)..(8)
<223>  Xaa is

<400>  5

Xaa Gln Trp Ala Val Gly His Xaa Leu

1               5


<210>  6
<211>  9
<212>  PRT
<213>  Artificial Sequence

<220>
<223>  Analogs of C-termional bombesin fragments


<220>
<221>  MISC_FEATURE
<222>  (1)..(1)
<223>  Xaa is D-Phe

<220>
<221>  MISC_FEATURE
<222>  (8)..(8)
<223>  Xaa is Cha

<220>
<221>  MISC_FEATURE
<222>  (9)..(9)
<223>  Xaa is Nle

<400>  6

Xaa Gln Trp Ala Val Gly His Xaa Xaa
1               5


<210>  7
<211>  9
<212>  PRT
<213>  Artificial Sequence

<220>
<223>  Analogs of C-terminal bombesin fragments


<220>
<221>  MISC_FEATURE
<222>  (1)..(1)
<223>  Xaa is D-Phe

<220>
<221>  MISC_FEATURE
<222>  (6)..(6)
<223>  Xaa is NMe Gly

<220>
<221>  MISC_FEATURE
<222>  (8)..(8)
<223>  Xaa is Sta

<400>  7

Xaa Gln Trp Ala Val Xaa His Xaa Leu
1               5

<210>  8
<211>  8
<212>  PRT
<213>  Artificial Sequence

<220>
<223>  Analogs of C-terminal bombesin fragments

<400>  8

Gln Trp Ala Val Gly His Leu Met
1               5


<210>  9
<211>  9
<212>  PRT
<213>  Artificial Sequence

<220>
<223>  Analogs of C-terminal bombesin fragments


<220>
<221>  MISC_FEATURE
<222>  (1)..(1)
<223>  Xaa is D-Phe

<220>
<221>  MISC_FEATURE
<222>  (6)..(6)
<223>  Xaa is NMeGly

<220>
<221>  MISC_FEATURE
<222>  (9)..(9)
<223>  Xaa is Nle

<400>  9

Xaa Gln Trp Ala Val Xaa His Leu Xaa
1               5


<210>  10
<211>  13
<212>  PRT
<213>  Homo sapiens

<400>  10

Gln Leu Tyr Glu Asn Lys Pro Arg Arg Pro Tyr Ile Leu
1               5                   10


<210>  11
<211>  6
<212>  PRT
<213>  Homo sapiens

<400>  11

Arg Arg Pro Tyr Ile Leu

1               5


<210>  12
<211>  8
<212>  PRT
<213>  Homo sapiens

<400>  12


<210>  13
<211>  8
<212>  PRT
<213>  Homo sapiens


<220>
<221>  MOD_RES
<222>  (2)..(2)
<223>  SULFATATION

<400>  13


<210>  14
<211>  8
<212>  PRT
<213>  Homo sapiens


<220>
<221>  MOD_RES
<222>  (3)..(3)
<223>  Nle

<220>
<221>  MOD_RES
<222>  (6)..(6)
<223>  Nle

<400>  14

Asp Tyr Xaa Gly Trp Xaa Asp Phe
1               5


<210>  15
<211>  8
<212>  PRT
<213>  Homo sapiens


<220>
<221>  MOD_RES
<222>  (2)..(2)
<223>  SULFATATION

<220>
<221> MOD_RES
<222> (3)..(3)
<223> Nle

<220>
<221> MOD_RES
<222> (6)..(6)
<223> Nle

<400> 15

Asp Tyr Xaa Gly Trp Xaa Asp Phe
1               5


<210> 16
<211> 9
<212> PRT
<213> Homo sapiens


<220>
<221> MOD_RES
<222> (1)..(1)
<223> Glu is D-Glu

<220>
<221> MOD_RES
<222> (2)..(2)
<223> Glu is from 0 to 10

<400> 16

Glu Glu Ala Tyr Gly Trp Met Asp Phe
1               5


<210> 17
<211> 8
<212> PRT
<213> Homo sapiens


<220>
<221> MOD_RES
<222> (5)..(5)
<223> Trp is D-Trp

<220>
<221> MOD_RES
<222> (6)..(6)
<223> X is N-MeLeu

<400> 17

Asp Tyr Met Gly Trp Xaa Asp Phe
1               5

## Description

### Summary of the invention

The invention concerns the obtainment, by using a kit, of liposomal doxorubicin in which liposomes are externally modified with a targeting peptide able to drive liposomal doxorubicin in a selective way on membrane receptors over expressed in tumours.

### State of the art

Doxorubicin in liposomes (Doxil®, Caelix® in Europe) is a pegylated liposomal formulation of Doxorubicin, widely used in tumour therapy. Doxil/Caelix is an example of successful passive targeting of liposomes to tumors, it has much lower cardio-toxicity and most other side effects than conventional doxorubicin. In fact, the liposomal shell prevents from cardiac toxicity of doxorubicin, while the presence of long PEG chains on the external liposome surface helps in increasing circulation time of the entire vehicle.

Overall Doxil improves patient compliance and quality of life. Doxil is approved by the FDA (1995) and worldwide (as Caelyx) for:
- AIDS-related Kaposi's sarcoma (KS), Nov, 1995
- Relapsed ovarian cancer, 1999; after platinum-based treatment, 2005
- Metastatic breast cancer with cardiac risk, Europe, 2003
- Multiple myeloma in combination with VELCADE® (bortezomib), 2007.

Doxil is based on 2 patent families (1988/1989) those of Liposome Technology Inc. (LTI) on the pegylated liposomes, and those of Yissum (Barenholz and Haran) on drug loading Licensed to LTI which was acquired by ALZA which was acquired by J & J. Both patent families were expired before April 2014. Doxil is was produced by Ben Venue Laboratories in the United States for Janssen Products LP, a subsidiary of Johnson & Johnson for global distribution. 2011 onward Doxil Shortage, August 2013 Doxil production stopped.

In February 2013 FDA approved the Lipodox of Sun Pharma as first generic Doxil.

Other companies are developing new generic Doxil, but they have not been yet approved, in most case for their low quality.

DOXIL (doxorubicin HCl liposome injection) is doxorubicin hydrochloride (HCl), an anthracycline topoisomerase II inhibitor, that is encapsulated in STEALTH® liposomes for intravenous use.

The chemical name of doxorubicin HCl is (8S,10S)-10-[(3-amino-2,3,6-trideoxy-α-L-lyxohexopyranosyl)oxy]-8-glycolyl-7,8,9,10-tetrahydro-6,8,11-trihydroxy-1-methoxy-5,12-naphthacenedione hydrochloride. The molecular formula is C₂₇-H₂₉ -NO₁₁•HCl; its molecular weight is 579.99.

DOXIL is a sterile, translucent, red liposomal dispersion in 10-mL or 25-mL glass, single use vials. Each vial contains 20 mg or 50 mg doxorubicin HCl at a concentration of 2 mg/mL and a pH of 6.5. The STEALTH liposome carriers are composed of cholesterol, 3.19 mg/mL; fully hydrogenated soy phosphatidylcholine (HSPC), 9.58 mg/mL; and N-(carbonyl-methoxypolyethylene glycol 2000)-1,2-distearoyl-sn-glycero-3-phosphoethanolamine sodium salt (MPEG-DSPE), 3.19 mg/mL. Each mL also contains ammonium sulphate, approximately 2 mg; histidine as a buffer; hydrochloric acid and/or sodium hydroxide for pH control; and sucrose to maintain isotonicity. Greater than 90% of the drug is encapsulated in the STEALTH liposomes. The clinical dose is 50 mg Doxorubicin/m², with successive administrations ever 4 weeks.

The drug is encapsulated, in crystalline form, in the liposome inner core, following its loading obtained through an ammonium gradient established between the inner core and the external medium. The drug/lipid ratio is 0.5. Liposomes are very stable both in solution and after in vivo injection, their dimensions are around 100 nm with very low polydispersity index.

Doxil/Caelix, their generic analogues, and all liposomal drugs already on the market or in development in clinical trials are not selectively targeted, and their accumulation on tumour cells is only due to the enhanced permeability and retention (EPR) effect, based on the higher permeability of the vessels around tumor sites.

The presence on the liposome external surface of active ligands able to recognize specific membrane receptors expressed, or over-expressed, by tumor cells, could improve doxorubicin accumulation only on target malignant cells, with reduction of severe adverse effects on non-target organs. Consequently, researchers focused their attention in the developing novel sophisticate liposome-based delivery systems able to enhance the therapeutic index of the encapsulated anticancer drugs by improving selective delivery by means of a targeting ligand on their surface, allowing the drug of reaching only intended target organs and cells at full concentration.

Target selective ligands of high interest are peptides able to bind with nanomolar affinity membrane receptors, such as G-Protein Coupled Receptors (GPCR) over expressed by cancer cells.

Very promising receptors and the related peptide ligands are:
i) the CCK-R receptors (CCK-1R and CCK-2R receptor subtypes) and the peptide cholecystokinin, in the full sequence, or as truncated peptide or as stable analogue;
   in fact, cholecystokinin receptors are over expressed by pancreatic tumours, neuroblastomas, and meningiomas (CCK-1R) or by gastric cancer, colon cancer, small cells lung cancer and medullary thyroid cancer (CCK-R2). The corresponding peptide ligands are the C-terminal cholecistokinin fragment CCK8 in its sulphated or non-sulphated form, in some cases having the Met residues substituted by more stable Norleucine residues; or by peptides known as gastrins or minigastrins.
ii) the Somatostatin receptors (sstr 1-5 subtypes) and the somatostain or octreotide peptide or their stable analogues;
   the five somatostatin receptors subtypes (sstr1, sstr2, sstr3, sstr4 and sstr5) are over expressed by Hodgkin and non-Hodgkin lymphomas, and in several types of breast cancer. The most known peptide ligand is the cyclic octapeptide Octreotide that displays high affinity towards sstr2 and sstr5.
iii) the Neurotensin receptor, the neurotensin peptide or its corresponding active fragments in monomeric or multimeric forms;
   in fact, neurotensin receptors NTR1, NTR2, NTR3 (Sortilin) are over expressed by the small cells lung tumour, by prostate carcinomas, and by colon and pancreatic tumours. The best ligands for these receptors are the neurotensin peptide with 13 amino acid residues having the sequence: QLYENKPRRPYIL, or its truncated form with sequence: RRPYIL, and tetrameric adducts having four copies of the active peptide ligand bound to a lysine scaffold.
iiii) the Gastrin Releasing Peptide Receptor (GRP) and the other BB1-4 bombesin receptors, the bombesin peptide or its stable, agonist or antagonist analogues;
   the bombesin receptor family consisting of four receptor subtypes (BB1, GRP, BB3 and BB4) are of interest as targets for diagnostic and therapeutic applications. Overexpression of the first two members of this family has been well documented in several human malignant tumors such as breast, prostate and ovarian cancer. The peptide ligand displaying high affinity towards these receptors is the bombesin peptide (BN) and its truncated C-terminal form with 9 amino acid residues. Bombesin analogues with higher in vivo stability and with antagonist properties have also been studied and developed.

In several cases the endogenous peptide ligand is substituted by corresponding truncated sequences that maintain receptor affinity but with an increased in vivo stability; or by new designed analogues to increase receptor affinity and acting as antagonist peptides.

Most of peptide ligands, both the endogeneous sequences or their analogues, are also used, after labelling with chelating agents and radioactive metal ions, as tracers in nuclear medicine to visualize tumour lesions overexpressing the target receptors. The most known example is Octreoscan, in which the octreotide peptide is derivatized with the DTPA chelating agent able to complex 111In(III) gamma emitting ion; it is used in clinical diagnosis and in imaging, to visualize tumours over expressing the somatostatin receptors.

Some of the above reported peptide ligands have been used for labelling of liposomal drugs in order to increase selectivity and specificity of the entire vehicle, and of the carried drug, on target cells.

Preparation of liposomes derivatized with a bioactive peptide such as CCK8, bombesin, octreotide, etc, has been initially described in WO 2006/128643.

A liposomal doxorubicin formulation containing, in the lipid mixture forming the liposome vehicle, a new synthetic amphiphilic monomer, Mon*Y*-BN is reported in WO 2013/046163. This new monomer contains two alkyl chains with 18 carbon atoms, a Peg spacer of 1500 Daltons, a Lys residue modified on the side chain with a chelating DTPA moiety, and a targeting peptide belonging to the bombesin family and showing nanomolar affinity to the GRP receptors.

Two examples of doxorubicin containing liposomes externally modified with bombesin (BN) peptide have been recently developed in order to selectively target gastrin releasing peptide (GRP) receptors overexpressed by several human tumors. These liposomes are based on the co-aggregation of 1,2-distearoyl-sn-glycero-3-phosphocholine (DSPC) phospholipid and BN containing amphiphilic monomers (Mon*Y*-BN) in 97/3 molar ratio. Firstly, the selective binding of BN-liposomes labeled with 111-Indium radioisotopes was evaluated *in vitro* by gamma counting. *In vivo* assays carried out on PC-3 xenograft-bearing mice injected intravenously with liposomal doxorubicin showed a higher tumor growth inhibition in the mice treated with DSPC/MonY-BN/DOX targeted liposomes compared to nonspecific DSPC/DOX liposomes (36%) and to the control animals (60%). However, the relatively short *in vivo* circulation time of [7-14]BN natural peptide (t_{1/2} = 15.5 h) suggested the authors to develop a new peptide analog BN-AA1, in which Leu¹³-Met¹⁴ C-terminal was replaced with Sta¹³-Leu¹⁴ and glycine residue was replaced with N-methyl-glycine for stabilization against aminopeptidase and carnitine enzyme, respectively. DSPC/MonY-BN-AA1/DOX liposomes showed a slightly greater extent than DSPC/MonY-BN/DOX liposomes.

In both cases, the obtained liposomes present diameter around 150 nm with a low polydispersity index (<0.2), the drug doxorubicin is loaded in peptide containing liposomes, just before their in vivo use, according to the standard procedure based on ammonium gradient and the final drug/lipid ratio is 0.5 identical to the Doxil/Caelix formulation.

However, the obtainment of this new class of liposome-based doxorubicin targeting vehicles presents some problematic issues both from chemical and formulative points of view. For example, it is very difficult and expansive the GMP production of the peptide containing monomer, Mon*Y*-BN with the high purity requested for liposome preparation; moreover doxorubicin loading should be performed just before the clinical use and it is not possible the production, and storage, of a finished "ready to use" vial containing the liposomal doxorubicin modified with bombesin peptide.

### Description of the invention

We have found that it is possible to overcome the above reported problematic issues by labelling the liposomal doxorubicin drug with the bioactive peptide after preparation of the liposomal doxorubicin. To do this, the invention is based on a **kit** containing a formulation of liposomal doxorubicin, a phospholipid modified with a reactive function (**compound 1**) such as DSPE-Peg-maleimide, in lyophilized form, and a peptide modified with an appropriate reactive function (**compound 2**) in lyophilized form and in stoichiometric 1:1 amount respect to compound 1.

The innovation of this invention concerns the obtainment of peptide modified doxorubicin liposomal drug trough two steps that should be easily performed by the clinicians just before the clinical use of the final product, starting from a pharmaceutical approved form of liposomal doxorubicin.

Many synthetic strategies have been established by modifying liposomes surfaces with targeting peptide sequences. The obvious goal of each approach is to achieve high coupling efficiency, leaving the bioactive peptide far away from the nanostructure surface and in its right specific conformation ready for binding to the target. The main difference between the different approaches concerns the method for introduction of the bioactive peptide on the aggregate surface: peptide should be introduced directly during nanostructure preparation by using a peptide amphiphile, according to a pre-functionalization strategy, or after nanostructures formulation by modifying their surfaces, with a post-functionalization method.

The first method, we used for preparation of bombesin labeled delivery systems of doxorubicin claimed in our patent WO 2013/046163, needs of a well purified amphiphilic peptide molecule; this molecule is mixed, in appropriate solvents and in the chosen ratio, with other amphiphilic molecules and phospholipids; liposomes are then obtained by evaporating the solvent or using extrusion procedures. After liposome preparation Doxorubicin is encapsulated by using the ammonium gradient method.

The second approach is based on liposomes decoration after their formulation. Peptide coupling after liposome preparation involves the introduction of suitable activated functional groups onto the external side of liposomes for covalent peptide binding. To guarantee correct orientation of the targeting ligand, bi-orthogonal and site-specific surface reactions are necessary. Functional groups commonly used are: a) amine for the amine-N-hydroxysuccinamide coupling method, b) maleimide for Michael addition, c) azide for Cu(I)-catalized Huisgen cycloaddition22-24 (CuAAC), d) biotin for not covalent interaction with avidin or triphosphines for Staudinger ligation25-26, and recently hydroxylamine for oxime bond. The synthetic strategy to adopt should be opportunely selected in order to increase the reproducibility and the yield of the coupling reaction.

The new method for the obtainment of peptide modified doxorubicin liposomal drug exposing a peptide on the external surface, we describe here and object of the present invention, is based on this second approach.

We use a ready-to-use vial of liposomal doxorubicin; in a first step, the sterile, translucent, red liposomal dispersion of the doxorubicin drug is used to hydrate a lipid film of DSPE-Peg-maleimide, or other phospholipid modified with a reactive function (**compound 1**), in order to obtain the post-insertion of the DSPE-Peg-maleimide, or of the other phospholipids used, in the preformed doxorubicin containing liposome vehicles. In the second step, the modified liposomal dispersion of doxorubicin reacts, through the chemical function (i.e.: maleimide) present on compound 1 and inserted in the liposome structure during the first step, with a modified peptide (**compound 2**) containing an appropriate chemical function (i.e.: thiol function).

By using the kit object of the present invention, and following procedures described in the instruction document provided together with the kit, the above described steps could be efficiently and easily performed by the clinicians just before the clinical use in the patient.

The kit object of the present invention could also contain an additional component based on the targeting peptide modified with a chelating agent able to complex radioactive metal ions for diagnostic and imaging purposes. In this case patients to be treated with the peptide modified liposomal doxorubicin could be selected on the basis of the over expression of the targeting receptors.

For example, in the case of liposome labelling with peptides belonging to the bombesin family in order to target cancer cells over expressing the GRP receptors, the most efficient peptide derivative could be: DOTA-Peg-DPhe-Gln-Trp-Ala-Val-NMeGly-His-Sta-Leu-NH2 (in which Peg is a polyethylene linker obtained by two condensed units of 8-amine-3,6-dioxaoctanoic acid).

### Detailed description of the invention

**The invention concerns the obtainment of liposomal doxorubicin exposing a targeting peptide on the liposome external surface, according to a very efficient new method based on a kit.**

The kit for the preparation contains:
a) The sterile, translucent, red dispersion of the liposomal doxorubicin drug;
b) A phospholipid modified with a reactive function (**compound 1**) in lyophilized form and in amount between 1% and 10% respect to the total lipid composition of the liposomes; better between 2% and 4%;
c) A peptide modified with an appropriate reactive function (**compound 2**), in a 1:1 stoichiometric amount respect to compound 1 and having the general formula:

   **R-L-P**

   in which:
   **R** is a residue containing a function able to react, giving a stable covalent bond, with one of the chemical functions present on **Compound 1.**
   **L** is absent or is oxoethylene linker with a MW comprised between 100 and 1000 dalton, and containing amine and carboxylic functions to form amide bonds with R and P, respectively.
      or
      **L** is an natural or non-natural amino acid residue in L or D conformation, or 2 or 3 amino acid residues sequentially linked.
   **P** is a peptide sequence able to target with high affinity toward receptors over expressed by tumor cells belonging to the bombesin peptide family, to cholecistokinin peptide family, to the somatostatin peptide family or to the neurotensin peptide family.

The kit object of this invention could also contain an additional component (d) consisting of the targeting peptide P modified with a chelating agent capable of complexing radioactive metal ions for diagnostic and imaging in vivo applications.

The kit components **a, b, c,** and, possibly, **d** could be contained in vials, containers, little bottles or in pre-filled syringes and are here identified as (**A**), (**B**), (**C**) and, possibly, (**D**).

The modified phospholipid (**compound 1**) is preferably a DSPE-PEG2000 (N-(polyethylene glycol 2000)-1,2-diasteroyl-sn-glycero-3-phosphoethanolamine) derivative modified with the introduction of reactive functions such as: maleimido, azide, dibenzocycloctyl, aldehyde, carboxy, pyridyldithiol propionate, succinyl or glutaryl groups.

Therefore, **compound 1** is preferably chosen among:
a) DSPE-PEG(2000)-Maleimide lipid;
b) DSPE-PEG(2000)-azide lipid;
c) DSPE-PEG(2000)-DBCO lipid (DBCO = dibenzocyclooctyl);
d) DSPE-PEG(2000)-aldehyde lipid;
e) DSPE-PEG(2000)-carboxilic acid lipid;
f) DSPE-PEG(2000)-PDP lipid (PDP = pyridyldithiol propionate);
g) DSPE-PEG(2000)-Succinyl lipid
h) DSPE-PEG(2000)-Glutaryl lipid
in which DSPE-PEG(2000) = N-(carbonyl-methoxypolyethylene glycol 2000)-1,2-distearoyl-sn-glycero-3-phosphoethanolamine

### In Compound 2 having the general formula R-L-P

The preferred **R** groups are:
- Cys or SH(CH₂)nCOOH (n in the range 1- 5) to react with the maleimide function present on Compound 1a or with PDP moiety present on compound If;
- Pra (Propargylglycine) or propyolic acid or DBCO to react with the azide function present on Compound 1b, according to click chemistry procedures;
- Azide to react with the DBCO moiety present on Compound 1c, according to click chemistry procedures;
- Amine to react with the carboxy function present on Compound 1e, 1g, or 1h.

**L** is absent or is a linker such as oxoethylene linker with a MW comprised between 100 and 1000 dalton, and containing amine and carboxylic functions to form amide bonds with R and P, respectively; **or**
**L** is an natural amino acid residue or a Beta-alanine residue, or 2 or 3 amino acid residues sequentially linked. If Lys, Orn, Glu, or Asp are present in the linker, they could also contain, on their side chain, a chelating agent such as DTPA (2,2',2",2"'-((((carboxymethyl)azanediyl)bis(ethane-2,1-diyl))bis(azanetriyl))tetraacetic acid), GluDTPA (*N,N*-Bis[2-[bis[2-(1,1-dimethylethoxy)-2-oxoethyl]-amino]ethyl]-L-glutamic acid), DOTA (1,4,7,10-tetraazacyclododecane-1,4,7,10-tetraacetic acid), NOTA (1,4,7-triazacyclononane-1,4,7-triacetic acid) for labeling with a radio-active isotope or with a paramagnetic ion such as Gd(III).

P is a peptide sequence able to interact with nanomolar affinity toward target receptors over expressed by tumor cells. P belongs to one of the following peptide families.
**1) Peptide P belongs to the bombesin family and is chosen among:**
- the wild-type sequence of bombesin;
- the C-terminal fragments of peptide bombesin containing 6-9 amino acidic residues;
- analogues (agonist or antagonist) of the C-terminal fragment of the bombesin peptide with a general formula:

   AA1-Gln-Trp-Ala-Val-AA2 -His-AA3-AA4 -NH₂

   wherein:
   AA1 is DPhe, D-Cpa (3-cyclopropyl-D-alanine), D-Tyr, D-Trp or is absent,
   AA2 is NMeGly, Gly or β-Ala
   AA3 is Leu, Cha (cycloesil-alanine), Sta (statine), Met or Nle (Norleucine).
   AA4 is Met, Leu or Nle (Norleucine).
Peptides are bound to L (or in absence of L, directly to R) through their amino function on the N-terminus with formation of an amide bond.
The preferred peptide sequences, containing natural or unnatural amino acid residues are reported in Table 1.

**Table 1:**

| # | **Preferred peptide sequences (P) in the Bombesin family** |
|---|---|
| SEQ ID 1 | -Gln-Trp-Ala-Val-Gly-His-Leu-Nle-NH₂ |
| SEQ ID 2 | -Gln-Trp-Ala-Val-Gly-His-Cha-Nle-NH₂ |
| SEQ ID 3 | -Gln-Trp-Ala-Val-Gly-His-Sta-Leu-NH₂ |
| SEQ ID 4 | -Gln-Trp-Ala-Val-NMeGly-His-Sta-Leu-NH₂ |
| SEQ ID 5 | -DPhe-Gln-Trp-Ala-Val-Gly-His-Sta-Leu-NH₂ |
| SEQ ID 6 | -DPhe-Gln-Trp-Ala-Val-Gly-His-Cha-Nle-NH₂ |
| SEQ ID 7 | -DPhe-Gln-Trp-Ala-Val-NMeGly-His-Sta-Leu-NH₂ |
| SEQ ID 8 | -Gln-Trp-Ala-Val-Gly-His-Leu-Met-NH₂ |
| SEQ ID 9 | -DPhe-Gln-Trp-Ala-Val-NMeGly-His-Leu-Nle-NH₂ |

**2) Peptide P belongs to the cholecystokinin family and is chosen among:**
- the wild-type sequence of CCK8 peptide;
- the wild-type sequence of CCK8 peptide having Tyr in the sulphated form;
- the wild-type sequence of CCK8 peptide having Tyr in the sulphated form or in non-sulphated form and having Norleucine residues in substitution of the Met residues;

- the C-terminal fragments of peptide cholecystokinin containing between 5 and 9 amino acidic residues;
- the sequence of the peptide gastrin containing 1 or more D or L Glu residues at the N-terminus;
- analogues (agonist or antagonist) of the C-terminal fragment of the cholecystokinin peptide.
Peptides are bound to L (or in absence of L, directly to R) through their amino function on the N-terminus with formation of an amide bond.
The preferred peptide sequences, containing natural or unnatural amino acid residues are reported in Table 2.

**Table 2**

| # | **Preferred peptide sequences (P) in the cholecystokinin family** |
|---|---|
| SEQ ID 1 | Asp-Tyr-Met-Gly-Trp-Met-Asp-Phe- NH₂ |
| SEQ ID 2 | Asp-Tyr(S04)-Met-Gly-Trp-Met-Asp-Phe- NH₂ |
| SEQ ID 3 | Asp-Tyr-Nle-Gly-Trp-Nle-Asp-Phe- NH₂ |
| SEQ ID 4 | Asp-Tyr(SO4)-Nle-Gly-Trp-Nle-Asp-Phe- NH₂ |
| SEQ ID 5 | DGlu-Gluₙ-Ala-Tyr-Gly-Trp-Met-Asp-Phe-NH₂ (n from 0 to 10) |
| SEQ ID 6 | Asp-Tyr-Met-Gly-DTrp-NMeLeu-Asp-Phe-NH₂ |

**3) Peptide P belongs to the somatostatin family and is Octreotide or its analogues with amino acidic sequences reported in Table 3:**

**Table 3**

| # | **Preferred peptide sequences (P) in the somatostatin family** |
|---|---|
| SEQ ID 1 | **Octreotide:** D-Phe-Cys-Phe-D-Trp-Lys-Thr-Cys-Thr(ol) Cys-Cys cyclization |
| SEQ ID 2 | **Lanreotide:** Beta-D-Nal-Cys-Tyr-D-Trp-Lys-Val-Cys-Thr-NH₂ Cys-Cys cyclization |
| SEQ ID 3 | **Tyr3-Octrotide:** D-Phe-Cys-Tyr-D-Trp-Lys-Thr-Cys-Thr(ol) Cys-Cys cyclization |
| SEQ ID 4 | **Tyr3-Octrotate:** D-Phe-Cys-Tyr-D-Trp-Lys-Thr-Cys-Thr-NH₂ Cys-Cys cyclization |
| SEQ ID 5 | **RC-160:** D-Phe-Cys-Tyr-D-Trp-Lys-Val-Cys-Trp-NH₂ Cys-Cys cyclization |

**4) Peptide P belongs to the neurotensin family and is selected among the following peptides**
- the peptide neurotensin with 13 residues and having the following sequence: QLYENKPRRPYIL
- the peptide neurotensin with 6 residues and having the following sequence: RRPYIL
- peptide QLYENKPRRPYIL in a tetrameric form linked to a Lysine scaffold having three lysine residues;
- peptide RRPYIL in a tetrameric form linked to a Lysine scaffold having three lysine residues;
- peptide QLYENKPRRPYIL in a dimeric form with the two peptides linked to the alpha and epsilon amine functions of a lysine residue;
- peptide RRPYIL in a dimeric form with the two peptides linked to the alpha and epsilon amine functions of a lysine residue;

The following examples clearly describe the new procedure and the use of the kit for the efficient preparation of the liposomal doxorubicin externally modified with a targeting peptide.

In particular, examples 1, 2, 3 describes procedures and analytical controls for the preparation, by using the kit object of this invention, of liposomal Doxorubicin having liposomes derivatized on their external surface with an analogue and antagonist of the bombesin peptide to target tumors overexpressing GRP receptors. The final product is usable for the treatment of ovarian and prostate cancers.

### Examples

### Example 1 - Post-insertion of Compound 1 in Doxil, Lipodox (or other approved analogues) liposomal drugs.

In this example, Compound 1, contained in Vial (B), is DSPE-Peg2000-maleimide. It is inserted in the amount of 2% w/w of the total phospholipids, by using a post-insertion method in preformed liposomes loaded with doxorubicin such as the Doxil/Caelix® drugs or the approved generic Lipodox® drug.

In detail, Vial (A) contains a sterile, translucent, red liposomal dispersion of the doxorubicin drug (Doxil or Lipodox, Volume 25.0 mL - Doxorubicin quantity 2.0 mg/mL corresponding to 50.0 mg). Vial (B) contains 32.3 mg of a GMP preparation of DSPE-Peg2000-maleimide, in lyophilized form (compound 1).

The procedure consists in the transferring of the entire content of Vial (A) into Vial (B). The final vial is placed on a shaker at room temperature and leaved to shake for 60 minutes. A complete dissolution of the lyophilized powder previously present into the vial is immediately observed. After shaking for 60 minutes, the complete insertion of DSPE-Peg2000-maleimide in preformed doxorubic containing liposomes could be confirmed by DLS experiments.

DLS measurements performed on a small aliquot of the solution should evidence the presence of liposomes with diameter around 100 nm and the absence of DSPE-Peg2000-maleimide pure micelles with a smaller diameter (below 50 nm).

### Example 2 - Reaction of Compound 2 with the product obtained in Example 1.

In this example the preparation of the final compound starting from the product obtained in Example 1 is described. In this example is used a peptide derivative (compound 2) belonging to the bombesin family, in particular a stable antagonist analogue of the 1-9 C-terminal sequence of Bombesin. In this way the final product, liposomal doxorubicin modified with the bombesin analogue peptide, will be able to target in a selective way GRP receptors over expressed in several human tumor and could be used for therapy of such tumors such as ovaric cancer.

The content of Vial B, obtained as described above, is transferred to Vial C containing compound 2. This vial contains 17.2 mg of a GMP preparation of a Bombesin analogue modified at N-terminus with a Cys residue, in lyophilized form. The preferred peptide sequence, used according to this example, is:
Cys-L-DPhe-Gln-Trp-Ala-Val-NMeGly-His-Sta-Leu-NH₂ in which L is NH-(CH₂CH₂O)₆-CH₂CH₂CO. The quantity of the peptide derivative (compound 2) (17.2 mg) is in 1:1 molar ratio with DSPE-Peg2000-maleimide (compound 1) inserted in liposomes according to the previously reported step (example 1). The vial is placed on a shaker at room temperature and leaved to shake for 30 minutes.

During the reaction time of 30 minutes a complete quantitative reaction is performed between the maleimide function present on the liposome external surface and sulphydryl function present on the peptide derivative. This guarantees on the absence of free peptide derivative in the final product.

### Example 3 - Characterization of the final product.

Characterization of the final product aims to determine: i) if all the peptide derivative has completely reacted with the maleimido function and, thus the absence of the free peptide in solution; ii) if dimensions and polydispersity index of the final liposomes are the same of the non modified product initially contained in Vial (A); iii) if more than 90% of the total Doxorubicin is still contained in liposomes as in the starting product.

To verify point i) the procedure consists in the following steps:
- Transfer 250 microliters from final solution present in vial (C) to a clean eppendorf and add 150 microliters of distilled water.
- Transfer the solution to a Sephadex G50 column (i.e.: a GE Healthcare - Illustra Nick Column) and elute with 800 microliters of water to obtain the liposomal fraction;
- Elute with additional 2.0 mL of water to obtain the free peptide fraction;
- Analysis by UV-Vis spectrometry: place in a 1.0 cm path length cell the free peptide fraction and read UV absorbance at 280 nm. The absence of free peptide is confirmed if Abs₂₈₀ < 0.016.
- Analysis by HPLC chromatography: perform HPLC analysis on the free peptide fraction by using the following conditions: Column C18 (i.e.: Jupiter - Phenomenoex 150x25); eluents: water/0.1 % TFA (A) and Acetonitrile/0.1% TFA (B); method from 5% (B) to 70% (B) in 10 min; UV revelation at 210 nm. The peptide derivative should eluate at 8.0 min. The absence of free peptide is confirmed in absence of the peak at 8.0 min or with a peak around 8.0 min having an area < 500.
- Analysis by fluoresce spectroscopy: analyze by fluorescence spectroscopy the liposomal fraction by exciting at 280 nm (excitation for the tryptophan residue) measuring fluorescence emission between 300 and 400 nm; an emission peak around 350 nm confirm the presence of the peptide on external surface of the liposomal drug.

To verify point ii) the procedure consists in the following steps:
- Transfer 250 microliters from final solution present in vial (C) to a clean eppendorf;
- Measure liposome dimensions by DLS; positive results are obtained if liposome diameter is 98 ± 15 nm with a polydispersity index of 0.20 ± 0.05.

To verify point iii) the procedure consists in the following steps:
- Transfer 100 microliters from the starting liposomal doxorubicin product present in vial (A) to a clean eppendorf and add 300 microliters of distilled water;
- Transfer the solution to a Sephadex G50 column (i.e.: a GE Healthcare - Illustra Nick Column) and elute with 800 microliters of water to obtain the liposomal fraction;
- Elute with additional 2.0 mL of water to obtain the free Doxorubicin fraction;
- Analysis by HPLC chromatography: perform HPLC analysis on the free doxorubicin fraction by using the following conditions: Column C18 (i.e.: Jupiter - Phenomenoex 150x25); eluents: water/0.1 % TFA (A) and Acetonitrile/0.1% TFA (B); method from 5% (B) to 70% (B) in 10 min; UV revelation at 210 nm. The free doxorubicin should eluate at 10.7 min. Measure the area of the peak.
- Perform the same operations starting from the final product present in Vial (C). The area of the peaks corresponding to free doxorubicin in the analysis of sample in the starting product (Vial A) and in the analysis of sample in final product (Vial C) should be comparable with an error of ± 20%.

## Claims

1. A kit containing:
a) a doxorubicin liposomal formulation;
b) a phospholipid modified with a reactive group **(compound 1)**, in the form of lipid or lyophilic film and in amounts ranging from 1% to 10% by weight with respect to the total lipid component, preferably from 2% to 4%;
c) a peptide modified with a suitable reactive function **(compound 2)**, in 1:1 stoichiometric amount to the compound 1 and with general formula:
**R-L-P**
in which:
**R** is a residue containing a chemical group capable of reacting to give a stable covalent bond, with one of the chemical groups present on **compound 1,**
**L** is absent or is an oxyethylene linker with molecular weight ranging from 100 to 1000 daltons, containing amine and carboxylic groups to form an amido bond with R and **P,** respectively;
or
**L** is a of natural o non-natural amino acid residue, in L or D conformation, or 2 or 3 amino acid residues sequentially bonded;
**P** is a peptide with target activity to the receptors overexpressed by tumour cells belonging to the bombesin, cholecystokinin, somatostatin or neurotensin families.

2. A kit according to claim 1 wherein the modified phospholipid (**compound 1**) is a derivative di N-(polyethylene glycol 2000)-1,2-distearoyl-sn-glycero-3-phosphoethanolamine functionalized with maleimido, azido, dibenzocyclooctyl, aldehyde, carboxylic, pyridyldithiol propionate, succinyl, glutaryl groups.

3. A kit according to claims 1 or 2 comprising a further component d) consisting of a peptide P modified with a chelating agent capable of complexing radioactive metal ions.

4. A kit according to one or more of claims 1 to 3 wherein each component a), b), c) and possibly d) is contained in vials, ampoules or pre-filled syringes.

5. A kit according to one or more of claims 1 to 4 wherein the compound 1 is N-(polyethylene glycol 2000)-1,2-distearoyl-sn-glycero-3-phosphoethanolamine maleimide (DSPE-Peg maleimide) and the component c) (compound 2) is a modified, antagonist analogue of the bombesin peptide selected from:
| | |
|---|---|
| SEQ ID 1 | R-L-Gln-Trp-Ala-Val-Gly-His-Leu-Nle-NH₂ |
| SEQ ID 2 | R-L-Gln-Trp-Ala-Val-Gly-His-Cha-Nle-NH₂ |
| SEQ ID 3 | R-L-Gln-Trp-Ala-Val-Gly-His-Sta-Leu-NH₂ |
| SEQ ID 4 | R-L-Gln-Trp-Ala-Val-NMeGly-His-Sta-Leu-NH₂ |
| SEQ ID 5 | R-L-DPhe-Gln-Trp-Ala-Val-Gly-His-Sta-Leu-NH₂ |
| SEQ ID 6 | R-L-DPhe-Gln-Trp-Ala-Val-Gly-His-Cha-Nle-NH₂ |
| SEQ ID 7 | R-L-DPhe-Gln-Trp-Ala-Val-NMeGly-His-Sta-Leu-NH₂ |
| SEQ ID 8 | R-L-Gln-Trp-Ala-Val-Gly-His-Leu-Met-NH₂ |
| SEQ ID 9 | R-L-DPhe-Gln-Trp-Ala-Val-NMeGly-His-Leu-Nle-NH₂ |
in which:
- **R** is: Cys o SH(CH2)nCOOH (n ranging from 1 to 5) to react with the maleimide group;
- **L** is absent or is an oxyethylene linker with molecular weight ranging from 100 to 1000 daltons, containing amino and carboxylic group to form an amido bond with R and the peptide, respectively;
or
L is a natural or non-natural amino acid residue, in L or D conformation, or 2 o 3 amino acid residues sequentially bonded.

6. A kit according to claim 5 wherein the L residue present in the bombesin peptide derivative comprises Lys, Orn, Asp or Glu residues functionalized with the chelating agents DTPA, GluDTPA, DOTA, NOTA, for the subsequent labelling with metal or paramagnetic ion radioactive isotopes.

7. A kit according to one or more of claims 1 to 6 wherein the peptide has the amino acid sequence Cys-L-DPhe-Gln-Trp-Ala-Val-NMeGly-His-Sta-Leu-NH₂ wherein L is NH-(CH₂CH₂O)₆-CH₂CH₂CO.

8. A kit according to one or more of claims 1 to 7 wherein the component d) is the DOTA-Peg-DPhe-Gln-Trp-Ala-Val-NMeGly-His-Sta-Leu-NH2 derivative wherein Peg corresponds to a polyoxyethylene linker resulting from two condensed 8-amino-3,6-dioxaoctanoic acid units.

9. Use of the kit of claims 1-8 for the preparation of a liposomal doxorubicin formulation wherein liposomes are externally modified with a target peptide.

10. Use according to claim 9 wherein the target peptide is stable antagonistic bombesin analogue.

11. A kit of claims 1-8 for use in the therapy of ovarian tumor, both first line and second line after the appearance of the resistance to platinum anticancer drugs.

12. A kit of claims 3-8 for use in the diagnosis and therapy of ovarian tumor or metastatic ovarian tumor wherein overexpression of GRP receptors is present, in which component d of the kit is used for selecting patients to be subjected to therapy with liposomal doxorubicin functionalized with a peptide of the bombesin family and monitoring the progression of the disease and the efficacy of the therapeutic treatment.
